Europäisches Patentamt

(19) European Patent Office (11) Publication number: **0 224 284**

Office européen des brevets **B1**

(12) **EUROPEAN PATENT SPECIFICATION**

(45) Date of publication of the patent specification: **23.05.90** (51) Int. Cl.⁵: **C07C 7/13**

(21) Application number: **86201800.9**

(22) Date of filing: **16.10.86**

(54) Process and apparatus for separating branched from unbranched hydrocarbons.   .

<table>
<tr><td>(30) Priority: <b>31.10.85 GB 8526812</b></td><td>(73) Proprietor: <b>SHELL INTERNATIONALE RESEARCH MAATSCHAPPIJ B.V., Carel van Bylandtlaan 30, NL-2596 HR Den Haag(NL)</b></td></tr>
<tr><td>(43) Date of publication of application: <b>03.06.87 Bulletin 87/23</b></td><td rowspan="2">(72) Inventor: <b>Sie, Swan Tiong, Badhuisweg 3, NL-1031 CM Amsterdam(NL)</b></td></tr>
<tr><td>(45) Publication of the grant of the patent: <b>23.05.90 Bulletin 90/21</b></td></tr>
<tr><td>(84) Designated Contracting States: <b>AT BE DE ES FR GB IT NL SE</b></td><td>(74) Representative: <b>Aalbers, Onno et al, P.O. Box 302, NL-2501 CH The Hague(NL)</b></td></tr>
<tr><td>(56) References cited: <br><b>EP-A- 0 006 665</b><br><b>FR-A- 1 538 661</b></td><td></td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process and an apparatus for separating branched- from unbranched hydrocarbons and further to a process for the isomerization of unbranched hydrocarbons.

It is known from FR-A 1 538 661 to separate branched- from unbranched hydrocarbons which are present in various oil fractions (e.g. hydrocarbon oil distillates) by contacting a gaseous mixture of said hydrocarbons with a bed of crystalline alumina silicate particles and removing unbranched hydrocarbons by contacting a fluidized bed, containing particles which have adsorbed unbranched hydrocarbons with a desorption gas such as steam and/or carbon dioxide, or methane.

However, according to such a known separation process unbranched hydrocarbons will not be obtained as such but in the form of a mixture with desorption gas, which will in many cases be unsuited for further use without additional separation treatment(s) such as absorption, fractionation or cooling.

Moreover, desorption gas molecules with a substantial affinity for the particulate adsorbent will have to be removed in an additional process step in order to retain at least a substantial part of the adsorption capacity of the adsorbent for further use in such a separation process.

It would be advantageous to avoid at least some of the aforementioned disadvantages of such a separation process whilst making optimum use of fluidized beds of particulate adsorbent in order to obtain hydrocarbon-containing products which can be directly used in other (e.g. hydroisomerization) processes without further separation- or other treatments.

It is also know from EP-A 6 665 to separate branched- from unbranched paraffins using a bed of molecular sieve in a process requiring at least four steps in order to effect satisfactory adsorption and desorption.

Surprisingly, it has now been found that mixtures comprising branched- and unbranched hydrocarbons can be separated into directly usable products by means of a process comprising only two steps.

Accordingly, the present invention provides a process for separating branched- from unbranched hydrocarbons which comprises the following steps:

(i) contacting a mixture comprising branched- and unbranched hydrocarbons with a fluidized bed comprising a particulate molecular sieve capable of selectively adsorbing unbranched hydrocarbons, and removing branched hydrocarbons therefrom; and

(ii) removing unbranched hydrocarbons from a fluidized bed comprising a particulate molecular sieve which has adsorbed unbranched hydrocarbons by contacting it with a gas containing molecular hydrogen.

In the process according to the invention fluidization is used in both steps, which makes it possible to use smaller amounts of molecular sieve than would be needed with the use of fixed- or moving beds, due to the improved mass- and heat transfer in fluidized beds. The present process can be carried out in a single fluidized bed wherein steps (i) and (ii) are operated sequentially, but it is preferred that step (i) is continuously carried out in an adsorption zone and step (ii) is continuously carried out in a separate desorption zone.

Suitably, steps (i) and (ii) of the process according to the invention are carried out at a substantially equal pressure from 1–100 bar abs., and preferably from 5–50 bar abs. In order to avoid the need for additional compression the operating pressure is preferably chosen such that hydrocarbon feed- and/or product streams can be used directly. Moreover, the temperatures in the process are preferably in line with the feed-temperatures and/or the temperatures at which a product stream is treated in a subsequent process, thus decreasing heat-exchanger requirements as much as possible.

The hydrocarbon feed mixture applied in step (i) of the process according to the invention suitably has a temperature from 50 to 400°C and preferably, from 150–300°C; most preferably the feed mixture is in the gas phase when it is contacted with particulate molecular sieve in step (i). To cope with the heat of adsorption means for heat transfer, e.g., in the form of cooling coils, may be installed in the fluidized adsorbent bed or the hydrocarbon feed mixture may be introduced at a lower temperature than the desired adsorption temperature, or both measures may be applied in combination.

Suitably, at least part of the feed mixture can be used to maintain the particulate molecular sieve in the adsorption zone(s) in a fluidized state by introducing said feed mixture at a space velocity of 0.1-10, and preferably 1-5 l (S.T.P.)/kg mol. sieve/s in the lower part(s) of the adsorption zone(s).

Various hydrocarbons comprising branched- and unbranched molecules can be separated by means of the process according to the invention, such as paraffinic-, olefinic- and/or substituted hydrocarbons (containing e.g. a halogen-, nitrogen-, oxygen- and/or sulphur moiety). The feed mixture may furthermore contain (un-)saturated cyclic hydrocarbons (e.g. cyclopentane, methylcyclopentane and/or benzene) in quantities of up to 30% by volume without having an adverse effect on the present process. A preferred feed mixture comprises branched- and unbranched- (i.e. normal) paraffins having 4-7 carbon atoms per molecule. Most preferably a feed mixture is used which comprises n-pentane, n-hexane and their isomers.

Preferably, step (ii) of the present process is carried out at a higher temperature than step (i) in order to attain a high desorption rate of unbranched hydrocarbons in the desorption zone. To sustain the temperature of desorption and to provide at least part of the heat of desorption of the unbranched hydrocarbons means for heat transfer, e.g., in the form of steam-heated coils, may be installed in the fluid bed, or the hydrogen-containing gas for desorption may be introduced at a high temperature, or both measures may be combined. The hydrogen-containing gas employed in step (ii) suitably has a temperature from 150-600 °C, and preferably from 250-450 °C.

Suitably, at least part of the hydrogen-containing gas employed in step (ii) is introduced in the lower part of a desorption zone at a space velocity of 0.2-20, and preferably 2-10 l (S.T.P.)/kg mol. sieve/s, sufficient to fluidize particulate molecular sieve present in said zone.

Hydrogen-containing gas can also be used to transport molecular sieve particles (which have adsorbed unbranched hydrocarbons), if needed from the adsorption- to the desorption zone(s) by introducing part of the gas in the adsorption zone near the inlet opening(s) of solids inlet means for the desorption zone. Alternatively said loaded molecular sieve particles can be transported by gravity flow from an upper adsorption zone to a lower desorption zone. Furthermore, hydrogen-containing gas can be advantageously used to prevent blockage of solids outlet means through which desorbed molecular sieve particles are returned from the desorption- to the adsorption zone(s) by introducing another part of the total amount of hydrogen-containing gas in the adsorption zone(s) near the outlet opening(s) of said solids outlet means.

The molecular hydrogen-containing gas to be used need not be completely pure and may e.g. contain up to 30 mol%, preferably not more than 20 mol%, of other substances, provided that they are substantially inert with respect to the feed and the applied molecular sieve.

The molecular sieves, which may be the same or different as employed in both steps (i) and (ii) of the process according to the invention must be selective with respect to the degree of branching of the hydrocarbons applied i.e. unbranched hydrocarbons should be substantially adsorbed, if possible only unbranched hydrocarbons, whereas, in order to attain good separation, branched hydrocarbons should not be retained in any substantial amount in the fluidized bed(s) of molecular sieve. Said selectivity is dependent to a large extent on the pore diameters of the molecular sieve, which diameters are preferably in the range from 0.3-0.8 nm, and most preferably from 0.4-0.6 nm. Suitably, carbon or synthetic or natural zeolites, such as 5A, erionite and offretite and preferably zeolite 5A, are used as molecular sieve.

Particles which can pass openings with a diameter from 10 μm to 10 mm, and preferably from 50 μm to 0.5 mm, can be suitably used in the fluidized bed(s) in the process according to the present invention; the particles which comprise molecular sieve material may in addition comprise a binder material such as alumina, silica or silica-alumina, in order to improve the crushing strength of the particles; said particles may also be mixed with particles which do not contain molecular sieve material. The particles may have any shape which is suitable for use in fluidized beds, such as cylinders or spheres, the latter shape being preferred due to its inherent abrasion strength.

Before being employed in the process according to the invention, the particulate molecular sieve is suitably at least partly dehydrated by heating at a temperature from 100-600 °C, depending on the type of material used. In some cases it is preferred to carry out the heating in situ in the process zone(s) in order to avoid contact of the molecular sieve material with moist air.

The invention further relates to an apparatus suitable for carrying out the process according to the invention, which comprises a housing enclosing an adsorption section having fluid inlet means arranged in its bottom part to maintain a fluidized bed of particulate solids therein and fluid outlet means arranged in the upper part of the adsorption section, and an enclosed desorption section having solids inlet means and separate outlet means extending downwardly from its bottom part into the adsorption section, fluid inlet means arranged in the bottom part of the desorption section to maintain a fluidized bed of particulate solids therein, and fluid outlet means arranged in its upper part.

Preferably said apparatus further comprises secondary fluid (e.g. hydrogen gas) inlet means arranged in the adsorption section near the lower end(s) of the solids inlet (or outlet) means of the desorption section.

The separation process according to the present invention is advantageously combined with a process for the isomerization of unbranched hydrocarbons which comprises contacting a feed containing unbranched hydrocarbons obtained in step (ii) of the separation process in the presence of a hydrogen-containing gas under hydroisomerization conditions with a hydroconversion catalyst in a hydroisomerization zone, separating at least part of the effluent from said zone into a mixture comprising branched- and unbranched hydrocarbons and a hydrogen-containing gas and subjecting at least part of said mixture to step (i) of the separation process.

At least part of the fresh feed for the isomerization process (which generally comprises a substantial amount of unbranched hydrocarbons) is suitably combined with the substantially unbranched hydrocarbons obtained in step (ii) of the separation process before entering the hydroisomerization zone. However, when the fresh feed contains a substantial amount of branched- and/or cyclic hydrocarbons which will usually not be hydroisomerized at the conditions prevailing in the hydroisomerization zone, it can be advantageous to combine at least part of said fresh feed with the hydrocarbons-containing part of the effluent from said zone and to subsequently introduce the combined feed in the adsorption zone, in order to obtain a stream substantially containing unbranched hydrocarbons which are subsequently hydroisomerized.

Hydrogen-containing gas which has been separated off from the effluent from the hydroisomerization zone in e.g. a flash separation zone is suitably recompressed and heated before being used in one or more zones of the integrated hydroisomerization/separation process as described hereinbefore. Fresh hydrogen-containing gas obtained from e.g. a reforming unit at elevated temperature and pressure is advantageously combined with said heated and recompressed hydrogen-containing gas in order to keep the size of the required compressor and heat-exchanger as small as possible. Suitably, a major part of the combined hydrogen-containing gas stream is led to the desorption zone (step (ii) of the separation process) in order to fluidize the molecular sieve particles present in said zone and to remove unbranched hydrocarbons from the particles. A minor part of said hydrogen-containing gas stream can be suitably employed to transport molecular sieve particles upwardly from the adsorption zone(s) to the desorption zone(s), whereas a further small amount of gas is preferably introduced in the adsorption zone near the solids outlet of the desorption zone. When required, additional hydrogen-containing gas can be introduced directly into the hydroisomerization zone.

The hydroisomerization step of the process according to the present invention is suitably carried out at a temperature from 100 to 300 °C, and preferably from 240 to 290 °C. The hydrogen partial pressure may vary between 3 and 70 bar, and preferably between 5 and 50 bar. The space velocity is suitably from 0.1 to 10, and preferably from 0.5 to 2 l liquid hydrocarbon feed/l catalyst/hour. The gas supply is suitably from 100 to 2500, and preferably from 400-1000, l (S.T.P.) pure hydrogen/kg hydrocarbon feed; the hydrogen/feed molar ratio is normally between 0.5:1 and 10:1, and preferably between 1:1 and 3:1.

The hydroconversion catalysts employed in fixed, fluidized-or moving beds in the hydroisomerization step are suitably specific hydroisomerization catalysts comprising one or more metals from group VIII of the Periodic Table of the Elements on a carrier material. The carrier material has acidic properties and may suitably consist of silica-alumina, in particular zeolites in the hydrogen form or exchanged with rare earth ions, or of alumina rendered acidic by combination with halogen (e.g. chlorine or fluorine). Preferably, the employed catalysts comprise at least one noble metal from group VIII (in particular platinum) on H-mordenite as carrier material. Most preferably, the H-mordenite is prepared by treating mordenite one or more times with an aqueous solution of an acid (e.g. hydrochloric acid) and, separately, one or more times with an aqueous solution of an ammonium compound (e.g. ammonium nitrate), followed by drying (e.g. at 100-200 °C) and calcining (e.g. at 400-700 °C) of the treated mordenite.

The (noble) metal(s) may be incorporated into the H-mordenite by any method known in the art, such as impregnation, precipitation or, preferably, ion exchange. If desired, the mordenite can be mixed with an inert binder before or after incorporation of catalytically active metal(s). Alternatively, the metal(s) may be incorporated into the binder before mixing with the mordenite. Suitable binders are e.g. natural clays (such as kaolin or bentonite) and refractory oxides such as alumina, silica, boria, chromia and zirconia or combinations thereof.

The isomerization catalyst particles may have any suitable form, such as tablets, spheres, granules or cylinders. The particles suitably have a diameter from 0.1-10 mm, and preferably from 0.5-5 mm.

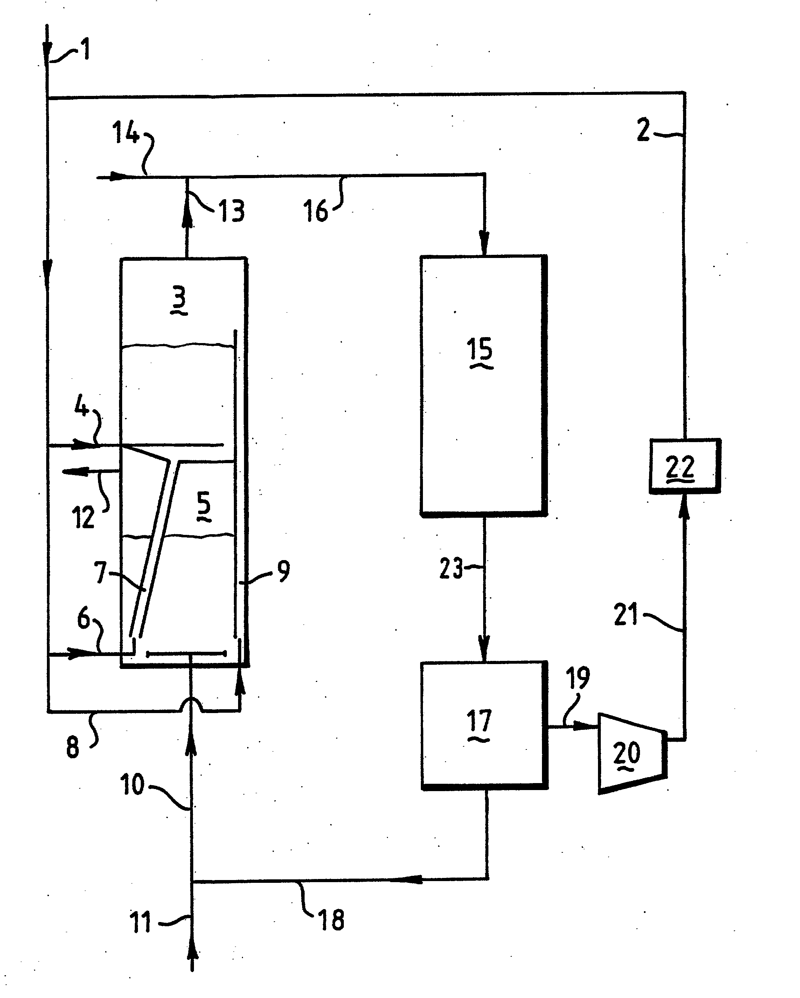

The invention is illustrated with the use of the Figure wherein a specific embodiment is depicted to which the present invention is by no means limited.

Fresh hydrogen-containing gas (stream (1)) is combined with the recycled hydrogen-containing gas stream (2), introduced in desorption zone (3) through line (4) and introduced into the bottom section of adsorption zone (5) through line (6) ending near the solids outlet (7) of zone (3) and introduced through line (8) into the solids inlet (9) of zone (3). A mixture of unbranched- and branched hydrocarbons is introduced into adsorption zone (5) through line (10), together with fresh hydrocarbon feed stream (11). A product stream mainly comprising branched hydrocarbons is withdrawn from adsorption zone (5) through line (12). A stream comprising unbranched hydrocarbons is withdrawn from desorption zone (3) through line (13), optionally combined with fresh hydrocarbon feed stream (14), and introduced into hydroisomerization zone (15) through line (16). The products from zone (15) are led via line (23) to separation zone (17) from which a hydrocarbon mixture is withdrawn through line (18) and may be recycled to line (10), whereas a separated hydrogen-containing gas stream, optionally in combination with fresh hydrogen-containing gas, is introduced through line (19) into compressor (20). Pressurized hydrogen-containing gas is led via line (21) to heating zone (22) and subsequently used as described hereinbefore.

## Claims

1. Process for separating branched- from unbranched hydrocarbons which comprises the following steps:

   (i) contacting a mixture comprising branched- and unbranched hydrocarbons with a particulate molecular sieve capable of selectively adsorbing unbranched hydrocarbons, and removing branched hydrocarbons therefrom; and

   (ii) removing unbranched hydrocarbons from particulate molecular sieve which has adsorbed unbranched hydrocarbons by contacting it with a gas;

   characterized in that steps (i) and (ii) both employ a fluidized bed comprising said particulate molecular sieve and a gas containing molecular hydrogen is used in step (ii).

2. Process according to claim 1 wherein step (i) is continuously carried out in an adsorption zone and step (ii) is continuously carried out in a separate desorption zone.

3. Process according to claim 1 or 2 wherein steps (i) and (ii) are carried out at a substantially equal pressure from 1-100 bar abs., and preferably from 5-50 bar abs.

4. Process according to any one of the preceding claims wherein step (ii) is carried out at a higher temperature than step (i) with a hydrogen-containing gas having a temperature from 150-600°C, and preferably from 250-450°C.

5. Process according to any one of the preceding claims wherein a gaseous mixture comprising branched- and unbranched paraffins having 4-7, and preferably 5 or 6, carbon atoms per molecule is used as feed.

6. Process according to any one of claims 2–5 wherein hydrogen-containing gas is used to transport molecular sieve particles from the adsorption- to the desorption zone(s).

7. Apparatus suitable for carrying out the process according to any one of the preceding claims which comprises a housing enclosing an adsorption section (5) having fluid inlet means (6) arranged in its bottom part to maintain a fluidized bed of particulate solids therein and fluid outlet means (12) arranged in the upper part of the adsorption section, and an enclosed desorption section (3) having solids inlet means (9) and separate outlet means (7) extending downwardly from its bottom part into the adsorption section (5), fluid inlet means (4) arranged in the bottom part of the desorption section to maintain a fluidized bed of particulate solids therein, and fluid outlet means (13) arranged in its upper part.

8. Apparatus according to claim 7 which further comprises secondary fluid inlet means arranged in the adsorption section near the lower end(s) of the solids inlet means of the desorption section.

9. Process for the isomerization of unbranched hydrocarbons which comprises contacting a feed containing unbranched hydrocarbons obtained in step (ii) of a process according to any one of claims 1–6 in the presence of a hydrogen-containing gas under hydroisomerization conditions with a hydroconversion catalyst in a hydroisomerization zone, separating at least part of the effluent from said zone into a mixture comprising branched- and unbranched hydrocarbons and a hydrogen-containing gas and subjecting at least part of said mixture to step (i) of a process according to any one of claims 1–6.

10. Process according to claim 9 wherein at least part of the hydrogen-containing gas separated from the effluent of the hydroisomerization zone is employed in step (ii) of a process according to any one of claims 1–6.

**Patentansprüche**

1. Verfahren zum Trennen verzweigter Kohlenwasserstoffe von unverzweigten Kohlenwasserstoffen, welches die folgenden Schritte umfaßt:
   i) In-Kontakt-Bringen eines Gemisches, welches verzweigte und unverzweigte Kohlenwasserstoffe umfaßt, mit einem teilchenförmigen Molekularsieb, welches fähig ist, unverzweigte Kohlenwasserstoffe selektiv zu adsorbieren, und Entfernen der verzweigten Kohlenwasserstoffe aus dem Gemisch; und
   ii) Entfernen der unverzweigten Kohlenwasserstoffe aus dem teilchenförmigen Molekularsieb, welches die unverzweigten Kohlenwasserstoffe adsorbiert hat, durch In-Kontakt-Bringen dieses mit einem Gas;
   dadurch gekennzeichnet, daß in beiden Schritten (i) und (ii) ein Wirbelbett angewandt wird, welches das genannte teilchenförmige Molekularsieb umfaßt und daß ein molekularen Wasserstoff enthaltendes Gas im Schritt (ii) verwendet wird.

2. Verfahren nach Anspruch 1, worin der Schritt (i) kontinuierlich in einer Adsorptionszone ausgeführt wird und der Schritt (ii) kontinuierlich in einer getrennten Desorptionszone ausgeführt wird.

3. Verfahren nach Anspruch 1 oder 2, worin die Schritte (i) und (ii) bei einem im wesentlichen gleichen Druck von 1 bis 100 bar abs. und vorzugsweise von 5 bis 50 bar abs. ausgeführt werden.

4. Verfahren nach einem der vorherstehenden Ansprüche, worin der Schritt (ii) bei einer höheren Temperatur als der Schritt (i) mit einem Wasserstoff enthaltenden Gas mit einer Temperatur von 150 bis 600°C und vorzugsweise von 250 bis 450°C ausgeführt wird.

5. Verfahren nach einem der vorherstehenden Ansprüche, worin ein gasförmiges Gemisch, welches verzweigte und unverzweigte Paraffine mit 4 bis 7 und vorzugsweise 5 oder 6 Kohlenstoffatomen je Molekül umfaßt, als Einsatzmaterial verwendet wird.

6. Verfahren nach einem der Ansprüche 2 bis 5, worin Wasserstoff enthaltendes Gas verwendet wird, um Molekularsiebteilchen von der (den) Adsorptions- zu der (den) Desorptionszone(n) zu transportieren.

7. Zum Durchführen des Verfahrens nach einem der vorherstehenden Ansprüche geeignete Vorrichtung, welche ein Gehäuse umfaßt, das einen Adsorptionsabschnitt (5) mit einem in seinem Bodenteil angeordneten Fließstoffeinlaßmittel (6), um darin ein Wirbelbett aus teilchenförmigen Feststoffen aufrechtzuerhalten und einem im oberen Teil des Adsorptionsabschnittes angeordneten Fließstoffauslaßmittel (12), und einen eingeschlossenen Desorptionsabschnitt (3) mit einem Feststoffauslaßmittel (9) und einem getrennten Auslaßmittel (7), welches sich von seinem Bodenteil abwärts in den Adsorptionsabschnitt (5) erstreckt, einem im Bodenteil des Desorptionsabschnitts angeordneten Fließstoffeinlaßmittel (4), um darin ein Wirbelbett aus teilchenförmigen Feststoffen aufrechtzuerhalten und ein in seinem oberen Teil angeordnetes Fließstoffauslaßmittel (13), umfaßt.

8. Vorrichtung nach Anspruch 7, welche ferner ein sekundäres Fließstoffeinlaßmittel umfaßt, welches im Adsorptionsabschnitt nahe dem (den) unteren Ende(n) des Feststoffeinlaßmittels des Desorptionsabschnittes angeordnet ist.

9. Verfahren zur Isomerisierung von unverzweigten Kohlenwasserstoffen, welches das In-Kontakt-Bringen eines Einsatzmaterials, das im Schritt (ii) des Verfahrens nach einem der Ansprüche 1 bis 6 erhaltene unverzweigte Kohlenwasserstoffe enthält, in Gegenwart eines Wasserstoff enthaltenden Gases unter Hydroisomerisierungsbedingungen mit einem Hydrokonversionskatalysator in einer Hydroisomerisierungszone, das Trennen von mindestens einem Teil des ausströmenden Materials aus der genannten Zone, in ein Gemisch, welches verzweigte und unverzweigte Kohlenwasserstoffe enthält, und in ein Wasserstoff enthaltendes Gas und das Unterwerfen von mindestens einem Teil des genannten Gemisches unter Schritt (i) eines Verfahrens nach einem der Ansprüche 1 bis 6, umfaßt.

10. Verfahren nach Anspruch 9, worin mindestens ein Teil des vom ausströmenden Material der Hydroisomerisierungszone abgetrennten Wasserstoff enthaltenden Gases im Schritt (ii) eines Verfahrens nach einem der Ansprüche 1 bis 6 angewandt wird.

## Revendications

1. Procédé pour séparer des hydrocarbures à chaîne ramifiée d'hydrocarbures à chaîne droite, qui comprend les étapes suivantes:

(1) mise en contact d'un mélange comprenant des hydrocarbures à chaîne ramifiée et à chaîne droite avec un tamis moléculaire en particules capable d'absorber sélectivement les hydrocarbures à chaîne droite, et séparation des hydrocarbures à chaîne ramifiée; et

(2) enlèvement des hydrocarbures à chaîne droite du tamis moléculaire en particules qui a des hydrocarbures à chaîne droite adsorbés par mise en contact de ce tamis avec un gaz;

caractérisé en ce que les étapes (1) et (2) utilisent toutes deux un lit fluidisé comprenant ledit tamis moléculaire en particules et qu'un gaz contenant de l'hydrogène moléculaire est utilisé dans l'étape (2).

2. Procédé selon la revendication 1, dans lequel l'étape (1) est conduite de manière continue dans une zone d'adsorption et l'étape (2) est conduite de manière continue dans une zone de désorption séparée.

3. Procédé selon la revendication 1 ou 2, dans lequel les étapes (1) et (2) sont conduites à une pression substantiellement égale comprise entre 1 et 100 bars abs et de préférence entre 5 et 50 bars abs.

4. Procédé selon l'une quelconque des revendications précédentes, dans lequel l'étape (2) est conduite à une température plus élevée que l'étape (1) avec un gaz contenant de l'hydrogène ayant une température de 150–600°C et de préférence de 250–450°C.

5. Procédé selon l'une quelconque des revendications précédentes, dans lequel on utilise comme charge un mélange gazeux de paraffines à chaîne ramifiée et à chaîne droite ayant 4–7 et de préférence 5 ou 6 atomes de carbone par molécule.

6. Procédé selon l'une quelconque des revendications 2–5, dans lequel du gaz contenant de l'hydrogène est utilisé pour transporter des particules de tamis moléculaire de la zone ou des zones d'adsorption à la zone ou aux zones de désorption.

7. Appareil utilisable pour la mise en œuvre du procédé selon l'une quelconque des revendications précédentes, qui comprend un logement délimitant une section d'adsorption (5) ayant des moyens d'entrée pour fluides (6) disposés dans sa partie inférieure de manière à maintenir un lit fluidisé de matières solides en particules et des moyens de sortie pour fluides (12) disposés dans la partie supérieure de la section d'adsorption, et une section de désorption enfermée (3) ayant des moyens d'entrée pour particules solides (9) et des moyens de sortie séparés (7) s'étendant vers le bas à partir de sa partie inférieure dans la section d'adsorption (5), des moyens d'entrée pour fluides (4) disposés dans la partie inférieure de la section de désorption de manière à y maintenir un lit fluidisé de matières solides en particules et des moyens de sortie pour fluides (13) disposés dans sa partie supérieure.

8. Appareil selon la revendication 7, qui comprend en outre des moyens d'entrée pour fluide secondaire disposés dans la section d'adsorption près de l'extrémité ou des extrémités inférieures des moyens d'entrée pour particules solides de la section de désorption.

9. Procédé d'isomérisation d'hydrocarbures à chaîne droite, qui comprend la mise en contact d'une charge contenant des hydrocarbures à chaîne droite obtenus dans l'étape (2) d'un procédé selon l'une quelconque des revendications 1–6 en présence d'un gaz contenant de l'hydrogène dans des conditions d'hydro-isomérisation avec un catalyseur d'hydroconversion dans une zone d'hydro-isomérisation, la séparation d'au moins une partie de l'effluent de cette zone de manière qu'on obtienne un mélange comprenant des hydrocarbures à chaîne droite et à chaîne ramifiée et un gaz contenant de l'hydrogène et l'exposition d'au moins une partie de ce mélange à l'étape (1) d'un procédé selon l'une quelconque des revendications 1–6.

10. Procédé selon la revendication 9, dans lequel au moins une partie du gaz contenant de l'hydrogène séparé de l'effluent de la zone d'hydro-isomérisation est utilisée dans l'étape (2) d'un procédé selon l'une quelconque des revendications 1–6.